# EUROPEAN PATENT SPECIFICATION

(11) **EP 4 041 166 B1**
(45) Date of publication and mention of the grant of the patent: **26.03.2025**
(21) Application number: 20793061.1
(22) Date of filing: 07.10.2020
(51) Int. Cl.: A61F 13/14

(54) **BREAST PAD AND METHOD OF MANUFACTURE**
BRUSTKISSEN UND VERFAHREN ZUR HERSTELLUNG
COUSSINET D'ALLAITEMENT ET PROCÉDÉ DE FABRICATION

(30) Priority: 09.10.2019 GB 201914628
(43) Date of publication of application: 17.08.2022
(73) Proprietor: Mayborn (UK) Limited, Newcastle upon Tyne, Tyne and Wear NE12 8EW (GB)
(72) Inventor: DIAMANT, Ben, Newcastle upon Tyne Tyne and Wear NE12 8EW (GB); LEWIS, Chris, Newcastle upon Tyne Tyne and Wear NE12 8EW (GB)
(74) Representative: Bloor, Sam
(86) International application number: PCT/GB2020/052476
(87) International publication number: WO 2021/069888

(56) References cited:
- EP-A1- 1 199 056
- FR-A1- 2 767 678
- GB-A- 904 580
- US-A- 6 074 272

## Description

### Technical Field of Invention

The present invention is concerned with breast pads used by pregnant or nursing women to absorb breast milk, as well as a method of manufacturing the breast pads. More particularly, but not exclusively, the invention relates to breast pads that are sized or are adaptable to conform to different shape breasts while maintaining absorbency.

### Background

Known breast pads are provided with a layer of absorbent material located between an outer, waterproof layer and an inner water-permeable layer capable of allowing liquid to wick through to the absorbent material. The layers and absorbent materials may each include one or more components, each component made from sheets of distinct substrates or a plurality of substrates mixed together.

US6074272 discloses a nursing pad liner having a layer of adsorbent material and a liquid impermeable outer layer for covering the outside of the adsorbent material and extending inside partially around the adsorbent material to form a reservoir.

GB904580 discloses a device for collecting unintentionally lost mother's milk consisting of an approximately cone-shaped hood of waterproof material and an apertured disc secured inside the hood to form a pocket with the hood.

FR2767678 discloses absorbent shields for breast during lactation including an impermeable supple support which is inserted between the breast and the brassiere.

Further components may be provided, such as adhesive patches on the outer surface of the outer layer to help hold the pad in position when worn by the user. Alternatively, or in addition, other substrates may be provided that work to distribute any absorbed liquid around the bulk of the absorbent material to prevent localised over-saturation.

Pads are typically manufactured in a flat configuration but are worn next to the skin under clothing or underwear in order to absorb breast milk that may leak from the nipple. Such flat pads will deform significantly in use when trying to conform to the user's body.

Pads that are deformed by wearing, and thus conform poorly to the shape of the breast, may be uncomfortable to wear. Without contacting the skin evenly, the effective absorbency of the pad may be reduced and breast milk may pool in spaces between the pad and the skin or leak around the pad. This causes discomfort and skin irritation as liquid is allowed to sit on the skin, or may cause leaks from the pad into clothing. Additionally, the flat shape of these pads can cause them to move away from the nipple (for example towards the armpit). When this occurs the coverage provided by the pad is reduced and it can be rendered ineffective.

Attempts have been made to improve the fit of breast pads and to provide the user with domed or 3-dimensional shaped pads from a flat disc. US5683286 discloses a pad with opposing pairs of radial cut-out portions in the absorbent material in order to provide shaping portions that enable the pad to fold from a flat pre-use configuration in a convex use configuration.

EP1943911B1 discloses the further step of bonding the inner faces of the inner layer together within the cut-out portions. The pad is thus provided with improved shape-retention properties.

On the other hand, the bonding of specific regions of the inner layer means that the user is only provided with a pad having a pre-determined fixed, convex configuration.

Further, both of the pads disclosed in the above two documents simply provide cut-out portions for the purpose of allowing the flat pre-use pad to deform to a convex shape which is symmetrical about a circumferential axis running through the pad at a perpendicular angle to the cut-outs themselves. However, it has been found that this shape is a poor imitation of a nursing woman's breast shape because it provides no account for the different shapes of an individual's left and right breasts (which are not symmetrical when worn in a bra), nor varying breast size within a population, nor the size variations of individual nursing women over the course of time.

Yet further, pads which include a fixed convex shape cannot adapt to being worn in different scenarios, for example first worn within a bra during the daytime when the user is generally upright, and then worn during night time when the user is generally lying down and particularly likely to move between different positions while sleeping. Plus, mothers' breasts change shape through their daily cycle and also between new-born and weaning stages.

It would be beneficial to users if they did not have to purchase multiple types of breast pad according to the different clothing and situations they encounter during a typical day.

### Summary

Accordingly, there is still a need for providing improved shapes which better conform to users' breasts and thereby provide less irritation when worn and more effective absorbency.

Aspects of the invention are set out in the claims.

A breast pad for nursing mothers is provided. The breast pad comprises an absorbent material having an asymmetric footprint, a liquid permeable front surface and a liquid impermeable back surface, the absorbent material arranged to absorb liquid that permeates the front surface. The absorbent material also comprises a body, a perimeter, and at least one cut-out portion extending inwardly from the perimeter into the body, wherein, the cut-out portion comprises two opposing edges each with a shaping portion. The absorbent material is adapted such that the perimeter around the absorbent material is a first length in a first configuration and, in a second configuration, the two shaping portions are positioned closer together such that the perimeter length is reduced and wherein the breast pad comprises an upper portion and a lower portion and wherein in the lower portion the absorbent material is adapted to have increased absorbent capacity.

In the first configuration the absorbent material may be planar. In this way, the breast pad is formed from sheets of substrates that are cut and assembled in an easy manner but which forms an asymmetric cone or cup once in a second configuration.

The asymmetric cone or cup may enclose a domed, asymmetrical volume. This occurs because a reduction in the perimeter pulls it to a smaller configuration thereby distorting the absorbent material and deforming it from a flat to a shape in a curved plane. The extent and degree of the distortion of the example breast pad ensures the absorbent material deflect by different amounts across a cross-section such that the degree of bending is not uniform when traversing a cross-section.

Depending on the shape of the asymmetric footprint, if cross-sections are taken across other orientations of the breast pad in its second configuration then no single cross-section would match any other.

In providing breast pads that can adapt to the individual shape of each user, or in providing a series of breast pads based on a single footprint, a single design of breast pad may be provided which can be used effectively by as wide a range as possible of nursing mothers. In other words, it may be possible to provide a footprint which may be useful for a number of breast pads appropriate to different bra cup sizes.

A further benefit is to provide a kit of more than one shape or size of a breast pad to provide the user with a selection of products that provide comfort and effective absorbency in many types of clothing and scenarios. Additionally, or instead of different shapes and sizes, a kit may provide breast pads with different absorbent capacities to enable the user to select a more absorbent pad when she is producing more milk, for example early in the morning.

A further benefit is that pads can be provided in pairs so that one is suitable for best conforming to a user's left breast and the other for conforming to the user's right breast.

A yet further benefit is to provide a pad that can be worn at night and adapt to the shape of a user's breast when lying down and the changing shapes that occur, for example when sleeping in different positions or at a time of day when more milk is produced.

Optionally, the two shaping portions may be provided at or adjacent to the perimeter. In this way, the mouth of the cut-out portion may be completely closed in the second configuration. In other words, the length of the portion of the perimeter in the mouth of a cut-out portion may be reduced to effectively zero.

Optionally, the two opposing edges may be flared as they approach the perimeter. In this way, the flared opposing edges may provide their own unique contribution to the domed shape because they provide an exaggerated deformation of the absorbent material in portions between the shaping portions and the perimeter.

Optionally, at least one of the front surface or back surface may form a deformable sheet that extends between the two opposing edges, wherein the deformable sheet may be configured to deform in the second configuration.

Optionally, the deformable sheet may form at least one pleat in the second configuration.

Further optionally, the deformable sheet may comprise securing means configured to position the two shaping portions in the second configuration.

Optionally, the securing means may be re-positionable. In this way, the user may adapt the breast pad to provide the best fit at all times. This is because, even once a nursing mother has selected an appropriate breast pad size from a range of sizes, each breast will change size while wearing the pad particularly leading up to, or just after, breastfeeding their infant or expressing milk. Consequently, by repositioning the securing means, the user can freely fine-tune the shape and size of the breast pad and always maintain the best fit.

The securing means may be provided on at least one of the front sheet, back sheet or deformable sheet. In this way, many types of fastener may be utilised and the fastener may be selected depending on whether the breast pad is single-use or washable and reusable.

Optionally, at least one of the front surface and the back surface may be provided as a sheet which is separable from the absorbent material.

Optionally, both the front and back surfaces are provided as sheets which extend beyond the perimeter and which together form a sealed edge portion. In this way, the absorbent material may be provided within a pocket and held in place by a small amount of bonding around the perimeter.

Optionally, the absorbent material may comprise more than one cut-out portion, each cut-out portion having two opposing edges and respective shaping portions.

Optionally, the absorbent material may be adapted such that in the second configuration the respective shaping portions each reduce the perimeter length by different amounts.

Optionally, the length that a first cut-out portion extends from the perimeter into the body may be greater than the length that a second cut-out portion extends.

Optionally, each cut-out portion may extend from the perimeter into the body along a different axis.

In these ways, the variability in possible asymmetric dome shapes is increased. Thus, the absorbent material may include a single, larger cut-out portion which makes a large reduction to the perimeter in the second configuration but which creates a significant deformation in the flat pad, thereby enclosing a volume with significant height.

Or, the several small cut-out portions may each make a contribution to the reduction in perimeter, but the deformation in the flat pad may not be as significant, thereby enclosing a shallower volume.

Or, again, the shaping portions can provide varying reductions of the perimeter and provide further options for asymmetrical dome shapes.

Optionally, the absorbent sheet may further comprise a liquid distribution portion arranged in contact with the front sheet. In this way, the liquid that permeates the front sheet may be more evenly distributed about the absorbent material, thereby preventing localised saturation and leaks.

The pad comprises an upper portion and a lower portion, wherein in the lower portion the absorbent material is adapted to have increased absorbent capacity. In this way, the absorbent material is better adapted should gravity cause liquid to collect towards to the base of the breast pad when worn by the user.

Optionally, the back sheet may comprise an adhesive portion suitable for securing the pad to the clothing of a user.

Optionally, the breast pad may be disposable.

Optionally, the asymmetric footprint may be a rounded convex quadrilateral.

Optionally, a set of two breast pads may be provided, wherein the asymmetric footprints of the two pads are mirror images of each other. In this way, the breast pads are best adapted to fit to the shape of a user's left and right breasts.

A method of manufacturing a breast pad for nursing mothers is provided. The method comprises: providing an absorbent material having an asymmetric footprint, a liquid permeable front surface and a liquid impermeable back surface, wherein the absorbent material comprises a body and a perimeter; arranging the absorbent material to absorb liquid that permeates the front surface; cutting the absorbent material to form at least one cut-out portion extending inwardly from the perimeter into the body, wherein, the cut-out portion comprises two opposing edges each with a shaping portion, and wherein the absorbent material is adapted such that the perimeter around the absorbent material is a first length in a first configuration; and deforming the absorbent material into a second configuration by positioning the two shaping portions closer together such that the perimeter length is reduced.

Optionally, deforming the absorbent material provides an asymmetric dome.

### Figures

Aspects of the invention are further described hereinafter with reference to the accompanying drawings, in which:
Figs. 1a and 1b illustrate examples of known breast pads with shapes to provide dome shaping;
Fig. 2a illustrates the dieline for a cutting tools used to cut the front and back sheets of a breast pad of a first aspect of the invention;
Fig. 2b illustrates the dieline for a cutting tool used to cut the absorbent sheet compatible with the sheets of Fig. 2a;
Fig. 2c illustrates the general quadrilateral shape of the dieline of Fig. 2a;
Fig. 3a-c illustrate the sheets cut with the dielines of Figs. 2a-c;
Fig. 4a-c illustrate a breast pad comprising the assembled sheets of Figs. 3a-c in a first, flat configuration;
Fig. 4d illustrates a breast pad the assembled sheets of Figs. 3a-c in a second configuration, shaped as an asymmetric dome;
Fig. 5 illustrates front and cross-sectional views of the breast pad of Fig. 4d;
Fig. 6 illustrates rear, top, bottom and side views of the breast pad of Fig. 4d;
Fig. 7a illustrates the absorbent sheet of a second aspect;
Fig. 7b illustrates the assembled sheets of the second aspect;
Fig. 8 illustrates the assembled sheets of a third aspect;
Fig. 9 illustrates an exploded view of the sheets of a fourth aspect;
Fig. 10a illustrates the assembled view of the adhesive strips of a fourth aspect;
Fig. 10b illustrates the assembled view of alternative adhesive strips;
Fig. 10c illustrates the use of release papers on adhesive strips;
Fig. 11 illustrates the assembled view of an alternative cut-out portions to the second aspect;
Fig. 12a illustrates the asymmetric footprint for a series of breast pads suitable for different cup sizes;
Fig. 12b illustrates the outline rectangular dimensions of the series of Fig. 12a;
Fig. 13a illustrates the asymmetric footprint for a further series of breast pads suitable for different cup sizes;
Fig. 13b illustrates the cut-out portion lengths of the series of Fig. 13a;
Figs. 14a-c illustrate cross-sectional views of the sub-layers of a series of absorbent sheets; and
Fig. 15 illustrates the assembled sheets of a breast pad with non-aligned axes arranged in a second configuration.

In the drawings, like reference numerals refer to like parts.

### Description of Examples

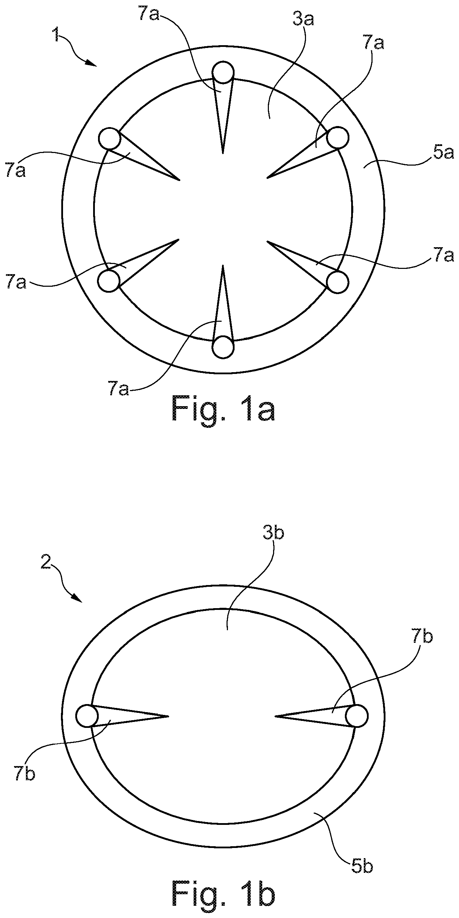

Figures 1a and 1b illustrate examples of known breast pads 1, 2 comprising multiple substrate layers. The substrate layers typically include absorbent sheets 3a, 3b sandwiched between at least two additional sheets which provide protective outer surfaces to the pads. The additional sheets cover each absorbent sheet 3a, 3b and extend beyond them to form sealed perimeter regions 5a, 5b thereby surrounding the absorbent sheets 3a, 3b.

In the examples shown in Figs. 1a and 1b, the absorbent sheets 3a, 3b are provided with at least one pair of diametrically opposed cut-out portions 7a, 7b comprising wedge-shaped regions where material has been removed from the absorbent sheets 3a, 3b. In contrast, the respective additional sheets do not have material removed and so extend to cover the cut-out portions.

In the example shown in Fig. 1a, the breast pad 1a is circular and provided with three pairs of cut our portions 7a. In this way, although the sheet is the manufactured from flat substrate sheets, the cut-out regions are thin enough to be folded and allow the pad to be shaped into a spherical dome.

In Fig. 1b, the breast pad 1b is an elongate ellipse and provided with a single pair of cut-out portions 7b. The pad is then folded diametrically along a fold line connecting the cut-out portions 7b and the inner surface of the fold are bonded together to form a dome which is symmetrical about at least two axes.

In contrast to the circular and elliptical sheets of known examples, the breast pads of a first embodiment are formed from sheets based on asymmetrical shapes. The breast pads are shaped and sized to be fit comfortably within a user's bra or clothing. They may be provided as a pair of matching, mirror-image pads so that one is suitable for the left breast and one for the right. The pads may be provided in pairs of matching sizes, as part of a range made up of several breast pads sizes.

The breast pad of an example is described below. It is sized and shaped to fit comfortably with bras with a cup size D or thereabout. This pad includes an absorbent sheet sandwiched between a liquid permeable front sheet and a waterproof back sheet. Each sheet is press-cut from a large substrate using a die-line as known in the art.

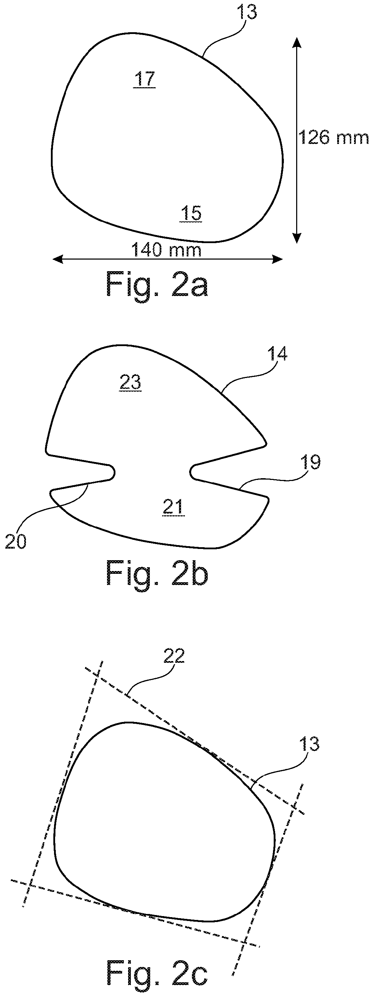

Fig. 2a shows a first die-line 13 of a cutting tool suitable for cutting both a front sheet and a back sheet for the same breast pad. The tool is asymmetrically shaped, with a broad lower portion 15 and an upper portion 17 that narrows to a rounded tip. The rounded tip is positioned slightly to one side of an axis running down the approximate centre of the die-line. In order to be appropriate for cup size D, the die-line is sized to fit into a rectangular area of 140mm wide by 126mm high.

A different die-line is used for cutting a compatible absorbent sheet. This second die-line 14 (Fig. 2b) is substantially the same asymmetrical shape as the first die-line 13 and is further provided with two wedge-shaped recesses 19 and 20. The recesses 19, 20 extend inwardly from the edge of the die-line 14 thereby dividing the die-line into its own lower portion 21 and upper portion 23.

Although substantially the same shape as the first die-line 13, the smaller footprint means that the second die-line is sized to fit into a rectangular area of 122mm wide by 111mm high.

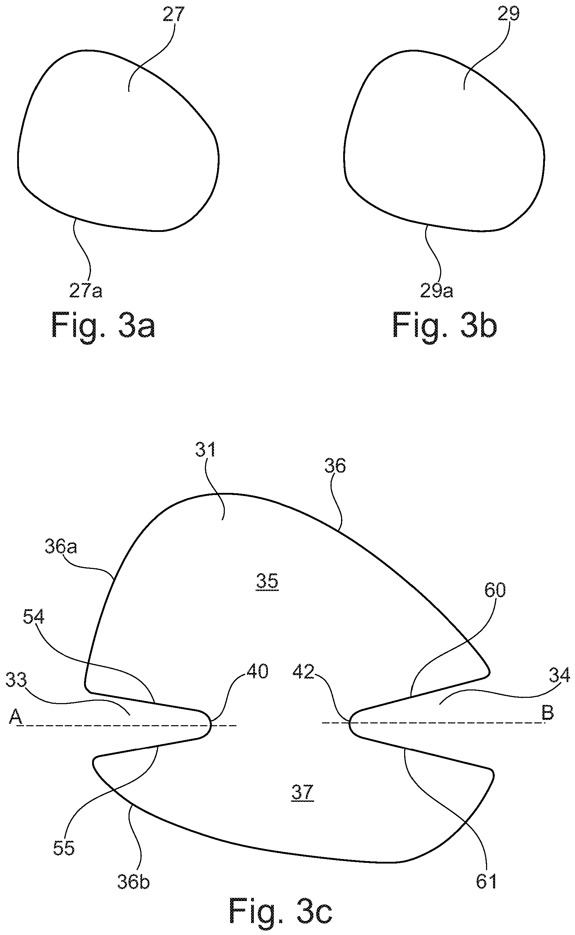

Using the first die-line 13, a front sheet 27 of the same asymmetrical footprint is press cut from a substrate (Fig. 3a). The front sheet 27 includes liquid permeable substrate comprising a hydrophilic non-woven polymer designed to allow moisture through in one direction (from breast to pad) so as to avoid any wetback. This substrate is capable of letting any breast milk that contacts the surface pass through, while having a soft, dry feel so that it is comfortable to the user's skin.

The first die-line 13 is also used to press cut a back sheet 29 with a footprint of the same size and shape as the front sheet 27 (Fig. 3b). The back sheet includes a waterproof substrate, for example Thermoplastic Polyurethane (TPU)film, which acts as a barrier to any moisture within a breast pad, preventing it passing through to the user's clothing and thus ensuring it is retained by the pad.

In common with the first die-line, both the front sheet 27 and the back sheet 29 each includes an asymmetric footprint including a perimeter 27a, 29a surrounding a main body of a broad lower portion and a narrowing upper portion. This footprint shape may be categorised as a rounded convex quadrilateral as it fits within the footprint of a convex quadrilateral, such as the quadrilateral 22 bounded by the broken line in Fig. 2c.

The second die-line 14 is suitable to press cut an absorbent sheet 31 of a matching footprint from a bulk substrate (Fig. 3c). In the example shown, the substrate includes a mixture of pulp fibre material combined with a water-absorbing polymer gel including a high absorbency material (or SAP) as is commonly known. A suggested SAP for use is Sodium Polyacrylate.

Due to the recesses 19, 20 the absorbent sheet 31 is formed with cut-out portions 33, 34 extending from the perimeter 36 into the body of the sheet, in other words towards the centre region of the sheet, thereby dividing the absorbent sheet 31 into an upper portion 35 and a lower portion 37. In this way, the perimeter 36 of the absorbent sheet 31 has two distinct perimeter sections 36a, 36b corresponding respectively to the section of perimeter that extends above (36a) and below (36b) the cut-out portions.

The first cut-out portion 33 includes two opposing edges. The opposing edges are an upper edge 54 that extends inwardly from the first perimeter section 36a, and a lower edge 55 that extends inwardly from the second perimeter section 36b. The edges 54, 55 tend towards a common axis A towards the centre of the body of the sheet 31. The edges meet at an inner point 40 located on axis A. In this way, the first cut-out portion 33 is substantially wedge-shaped with a mouth approximately 21mm wide and which extends 38mm horizontally into the sheet 31, and having straight edges mutually inclined by an angle of 21°.

The second cut-out portion 34 has aspects in common with the first, although it is not identical. In a similar manner to the first cut-out portion 33, the second cut-out portion 34 is substantially wedge-shaped, comprising edges 34a, 34b that tend towards a common axis B, to meet at an inner point 42 on the axis B, near the centre of the sheet 31. However, in contrast, the mouth is approximately 30mm wide and the cut-out 34 extends 44mm horizontally into the sheet 31. Furthermore, the edges 34a, 34b are not straight and so the mutual angle is not consistent over the full extent of the edges. Instead, they are mutually inclined by an angle of 31° (denoted B' in Fig. 3) near the inner point 42, but near the perimeter 36 they flare apart slightly.

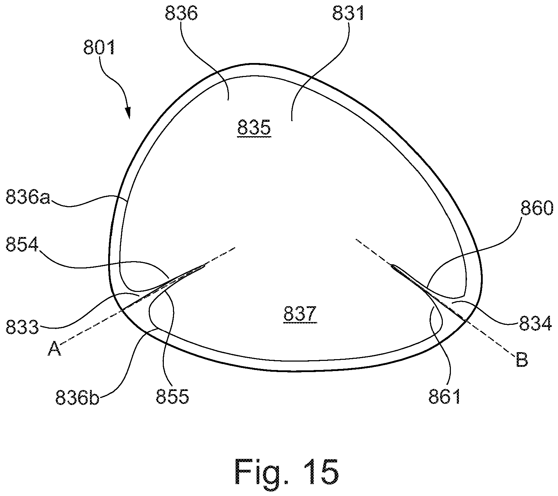

In the example shown in Fig. 3c, the axes A, B are very closely aligned however this is not always the case and other cut-outs can be chosen which extend into the body along axes in a different alignment, as shown in the breast pad 801 in Fig. 15a and 15b (described below).

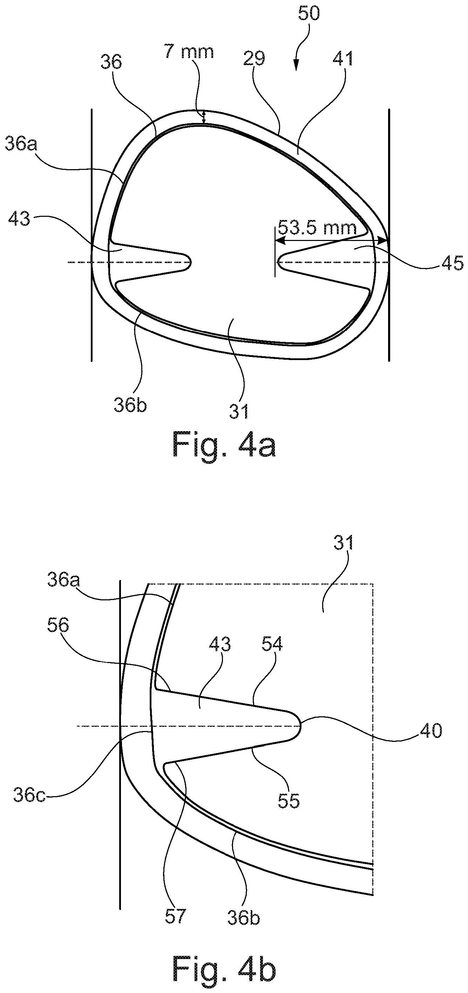

To assemble the breast pad, the sheets cut using the dielines are provided in a layered arranged. In the layered arrangement, the absorbent sheet 31 is positioned on top of the back sheet 29 such that the back sheet 29 overlaps out a short distance all around the perimeter 36 (Fig. 4a). The front sheet 27 (not shown in Fig. 4a) is positioned to exactly overlie the back sheet 29 thereby sandwiching the absorbent sheet between itself and the front sheet 27.

With the sheets positioned in layers, the overlapping portions of the front and back sheets are sealed together with an edge seal 41 approximately 7mm wide surrounding the perimeter 36 of absorbent sheet 31. The sealing may be provided by application of heat to form flat pad 50 with the same asymmetric footprint as the dieline 13. In this way, the flat pad 50 is provided with the absorbent sheet 31 trapped within a pocket formed by the front sheet 27, back sheet 29 and an edge seal 41.

As a consequence of the absorbent sheet 31 being sealed in a pocket and of the fact there are no cut-out portions in the front and back sheets, the region between the opposing edges of the cut-out portions 33, 34 is covered by the front and back sheets 27, 29. Because the front and back sheets include thin, pliable substrates then when the sheets are located in the region between the opposing edges they form deformable sheets 43 and 45.

In an alternative example (not shown) the absorbent layer may be integral with or adhered to one or both of the front and back sheets. In this way, the front and back sheets may be no longer separable from, in other words are integral with, the absorbent sheet. Such an arrangement might be advantageous in providing a breast pad which is increased dimensional stability because relative movement between the layers is eliminated. The arrangement may also allow for more efficient use of materials because a deformable sheet need only be made from one of the front or back sheet substrates. Further, the edge seal width (and therefore the required materials) may be minimised, for example to be approximately 4mm, 5mm or 6mm wide, because the increased dimensional stability negates the need to seal the absorbent sheet within a pocket.

In an alternative example, the absorbent layer is provided as a plurality of absorbent sub-layers. The absorbent sub-layers may all be the same substrate or may include different substrates in order to give different absorbency characteristics at different thicknesses through the absorbent layer. Thus, a central absorbent sub-layer in a series of three sub-layers may contain a higher concentration of SAP, surrounded by layers with a lower concentration, thereby providing a high-absorbency core. Alternatively, the sub-layers may be the same substrates but one may have a higher absorbent capacity simply by being thicker.

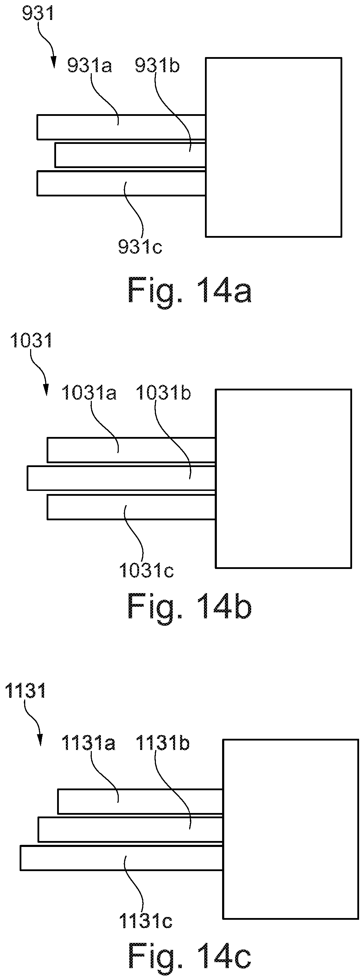

The absorbent sub-layers may also be provided as different sizes. For examples, as shown in cross-sectional view Fig. 14a, an absorbent sheet 931 comprises upper, lower and intermediary absorbent sub-layers (931a, 931c and 931b). The upper and lower sub-layers (931a, 931c) extend outward from the body of the pad to the perimeter of the absorbent sheet 931. However, intermediary sub-layer 931b does not extend as far. In this way, the absorbent sheet 931 is provided with a feathered perimeter that has a softer feel and aids comfort of the breast pad.

In an alternative arrangement, Fig. 14b, the intermediary sub-layer 1031b extends to the perimeter of the absorbent sheet 1031, while the upper and lower sub-layers 1031a, 1031c do not. Again, this provides feathered perimeter with softer feel and improved comfort.

In a further alternative arrangement, Fig. 14c, the lower sub-layer 1131c extends to the perimeter of absorbent sheet 1131 and the intermediary and top sub-layers 1131b, 1131a do not. Instead, the intermediary and top sub-layers extend progressively less to provide a stepped or angled edge. This edge will also have the softer feel and improved comfort and, further, may also flex more easily in the direction towards the lower sub-layer 1131c than it does towards upper sub-layer 1131a thereby having a soft, comfortable edge that resists deformation in a desired direction.

When used in breast pads, the absorbent sheets 931, 1031, 1131 may be used either way up. In other words, the top sub-layer may be positioned near the corresponding front sheet or the back sheet depending on the desired characteristics it may provide to the breast pad.

The physical aspects or composition of individual sub-layers may be modified in accordance with any of the alternatives described herein, to further alter the properties and perimeter of the absorbent sheet as a whole. Additional or fewer sub-layers may be used and the individual thicknesses of the sub-layers can be varied.

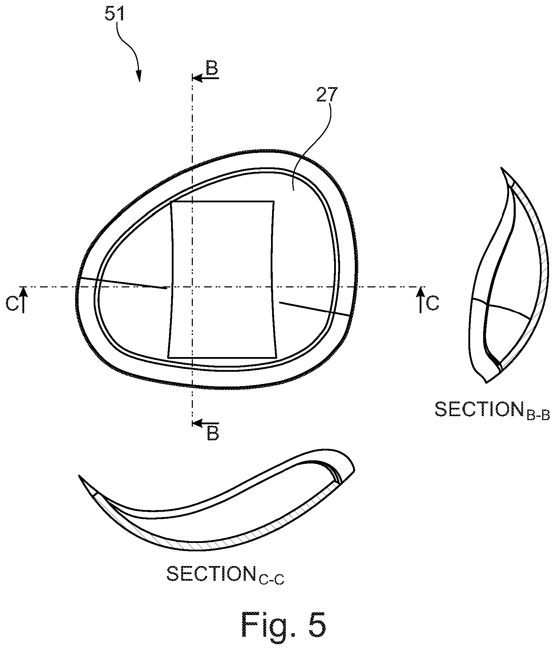

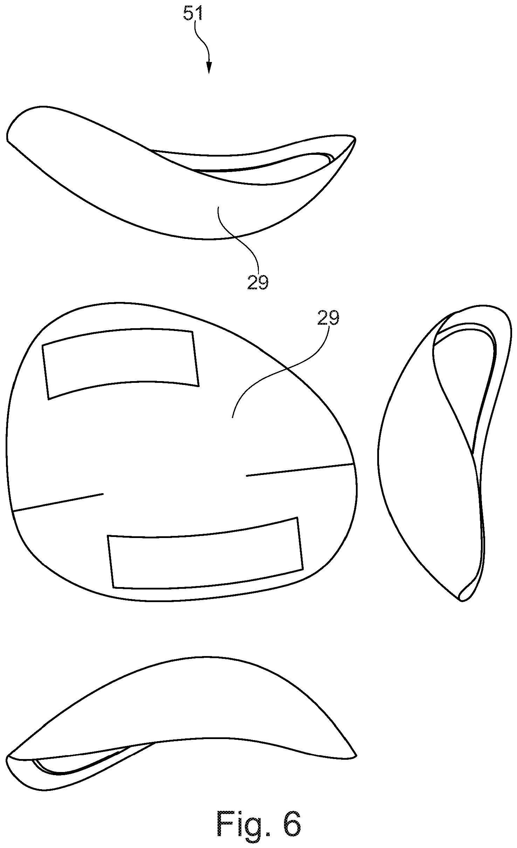

With the flat pad 50 fabricated, it is ready to be formed into a shaped breast pad 51. The details of the forming step are shown in Figs. 4b-4d and the resulting shaped breast pad 51 is shown in Figs. 5 and 6.

As explained above, the flat pad 50 includes an absorbent sheet 31 with a first cut-out portion 33 and a second cut-out portion 34. Each cut-out portion has opposing edges extending inwardly into the body of the absorbent sheet 31, eventually meeting on a respective axis, A or B. The opposing edges 54, 55 of the first cut-out portion 33 (Fig. 4b) each include a respective shaping portion 56, 57. In the flat pad configuration, the shaping portions 56, 57 are maximally spaced apart because the deformable sheet 43 therebetween is substantially flat. Accordingly, the distance between the first perimeter section 36a and the second perimeter section 36b at the mouth 36c of the cut-out portion 33 is also maximally spaced.

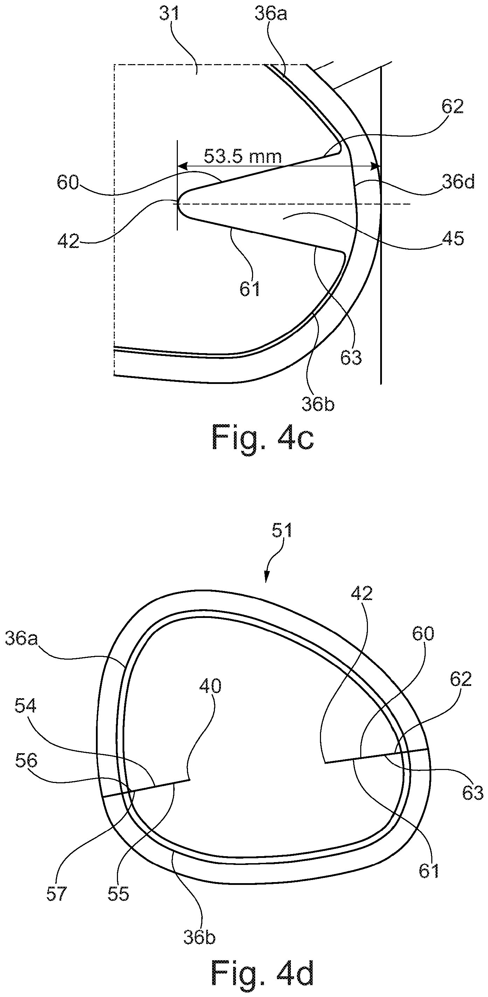

Similarly, the opposing edges 60, 61 of the second cut-out portion 34 (Fig. 4c), each include a respective shaping portion 62, 63 which, in the flat pad configuration, are maximally spaced apart. Accordingly, the distance between the first and second perimeter sections 36a, 36b at the mouth 36d of the cut-out portion 34 is also maximally spaced.

In this way, in the flat configuration the whole perimeter 36, including the gap between the first and second perimeter sections 36a, 36b, and mouths 36c, 36d is a first, maximal length.

It should be noted that due to the different lengths of the cut-out portions 34 and the angles that they form in the body of the absorbent material 31, then the mouths 36c and 36d in the example shown are not the same length. Depending on the chosen the lengths of the cut-out portions and angles that the edges are formed at, the lengths of the mouths may be substantially the same or they may be significantly different.

In forming the shaped breast pad 51 (Fig. 4d), the deformable sheet 43 is folded along the axis A with the back sheet 29 on the outside of the fold. In this way, the shaping portions 56, 57 are positioned closer together. Additionally, the deformable sheet 44 is folded along the axis B, again with the back sheet 29 on the outside of the fold, therefore bringing the shaping portions 62, 63 together. The respective edge seal portions are secured in order to hold each fold in place.

The folding of the deformable sheets 43, 43 and the sealing of their edge seal portions may be performed simultaneously, for example if the axes A and B overlap, by applying a heated press to both edge seal portions at the same time. Alternatively, for example if the axes A and B do not overlap, the edge seal portions may be folded and sealed one after another.

In the example shown, the edge seal portions are bonded across the full extent of the mouths 36c, 36d so that the respective pairs of shaping portions are brought permanently together. Thus, the lengths of the mouths 36c, 36d are reduced to effectively zero. The length of the perimeter 36 as a whole is correspondingly reduced.

In certain examples the bonding of the edge seal portions may be in addition to the bonding created when the edge seal 41 forms the flat pad 50. In other words, the edge seal 41 first forms a continuous seal around the absorbent sheet in the first configuration and, when the edge seal portions outside the mouths are bonded to form the second configuration, the edge seal portions are bonded for a second time.

More aptly however, the breast pad may be fixed in the second configuration more effectively if the edge seal 41 forms a discontinuous seal around the absorbent sheet in the first configuration, whereby no seal is created in the edge seal around the mouths of the cut-out portions. Consequently, when the edge seal portions outside the mouths are bonded to form the second configuration, they are bonded for a first time.

As a consequence of reducing the length of the perimeter 36, the breast pad 50 is deformed from a flat shape into a shaped breast pad 51 in the form of an asymmetric cone, enclosing a domed, asymmetrical volume. This occurs because a reduction in the perimeter pulls it into a smaller configuration, thereby distorting the absorbent material and deforming it from flat to a shape in a curved plane. Such a distortion is known and described in the prior art cited above. However, in those examples, the circular pad shape and matching pairs of cut-out portions only consider a symmetrical dome distortion. In contrast, the extent and degree of the distortion of the example breast pad 51 is illustrated in Figs. 5 and 6. Thus, in Fig.5 the surface of the front sheet 27 is shown along with the indicated cross-sections B-B and C-C. In this way, it is possible to see not only does the outer limits of the absorbent material deflect by different amounts across each cross-section, but the degree of bending is not uniform as you traverse either cross-section. Indeed, if other cross-sections were taken across other orientations then no single cross-section would match any other. This creates a unique shape which fits more comfortably than prior art pads.

The asymmetry of the deformation is also clear in Fig. 6, which shows the outer surface of the back sheet 29 viewed face on, as well as from above, below and the side.

Although the example above describes folding and heat-sealing to fix the breast pad in a shaped configuration, the shaping portions may be positioned together using other methods within the scope of the invention. For example, when the deformable sheet is folded, it may be folded with the front sheet on the outside of the fold. Alternatively, the deformable sheet may be folded repeatedly backwards and forward in the region of the edge seal to create a pleated edge with a resiliently expandable border.

Further alternatively, the breast pad may include an adhesive positioned within and securing a folded section.

Optionally, once any fold is fixed, the spare portion of the fold may be trimmed to avoid a sharp, folded corner.

Further alternatively, the shaping portions may be brought together in a manner which is adjustable, thereby allowing the user to fine-tune the size of the pad. For example, the surface of the front sheet may include small areas of hook and loop fasteners; the outer surface may include a strap and fastener; the edge seal portions may be clamped by a suitable clip or fastener; or the previously-mentioned adhesive may be repositionable. In either case, the degree to which the shaping portions have been brought together can be fixed by the user before using the pad and, optionally, unfixed then amended (to make the perimeter longer or shorter and thus reshape the breast pad) and then re-fixed.

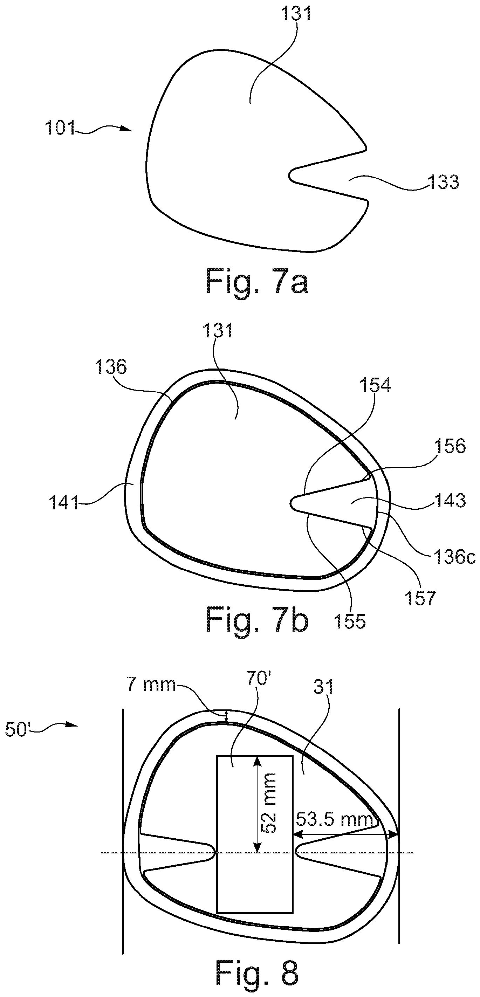

A further example is shown in Fig. 7a & 7b. In this example, the breast pad 101 includes absorbent material 131 pressed using a dieline with an asymmetric footprint, which has only one cut-out portion. Thus, as shown assembled in the flat configuration, the breast pad 101 includes only a single cut-out portion 133 with its own pair of opposing edges 154, 155 and their respective shaping portions 156, 157. The space between the opposing edges includes a deformable sheet 143 made from the extending front and back sheets. The front and back sheets also extend beyond the absorbent sheet perimeter 136 and are joined to form an edge seal 141.

In the same way as the first example, the perimeter 136 includes a mouth 136c spanning the gap in the absorbent material at the outer boundary of the cut-out portion 133. In the flat configuration, the mouth 136c has a length corresponding to the distance between the shaping portions 156, 157. However, the shaping portions 156, 157 can be brought together, significantly reducing the length of the mouth 136c and reducing the overall length of the perimeter 136, thereby distorting the absorbent material into an asymmetric dome (not shown).

In a further example shown in Fig. 15, a breast pad 801 is formed from an absorbent material 831 pressed using a dieline with two cut-out portions 833, 834 to form an upper portion 835 and a lower portion 837. In this example, the respective axes A, B of the cut-out portions 833, 834 are not in the same alignment. Nevertheless, as shown in Fig. 15, with the first pair of opposing edges 854, 855 and the second pair of opposing edges 860, 861 positioned together, the overall length of the perimeter 836 is reduced, again distorting the absorbent material 831 into an asymmetric dome.

In an example not shown, the breast pad may have an absorbent sheet with an asymmetric footprint comprising three or more cut-portions spaced around the perimeter and each extending inwardly into the body of the sheet. As with the other examples cited herein, the pairs of opposing edges would each have shaping portions that, once positioned closer together, would contribute to reducing the overall length of perimeter enabling the flat breast pad to form a new configuration in an asymmetric dome shape. Each cut-out portion would include opposing edges arranged to tend towards and meet a unique axis. Or, in other words, the axes of each cut-out portion would be at a different orientation.

In breast pads where the absorbent sheet has more than one cut-out portion, each cut-out portion may be unique or, it may match a counterpart. Thus, the configuration of any one cut-out portion is not dependent on the configuration on any other, That said, the manner in which each cut-out portion contributes to reducing the perimeter of the absorbent material may be taken into account in determining the overall asymmetric dome shape of the breast pad.

A yet further example is shown in Fig. 8 comprising a breast pad 50' with all the corresponding features of the breast pad 50. In this example, a liquid distribution portion 70' is positioned between a portion of the absorbent material 31 and the front surface (not shown). The liquid distribution portion 70' includes a hydrophilic non-woven sheet which receives liquid passing through the front surface. Once received, the liquid distribution portion 70' rapidly wets out across a large proportion of its surface area, enabling the liquid to spread out over a larger area of the absorbent material 31 and thereby minimise uneven absorption and storage of the liquid.

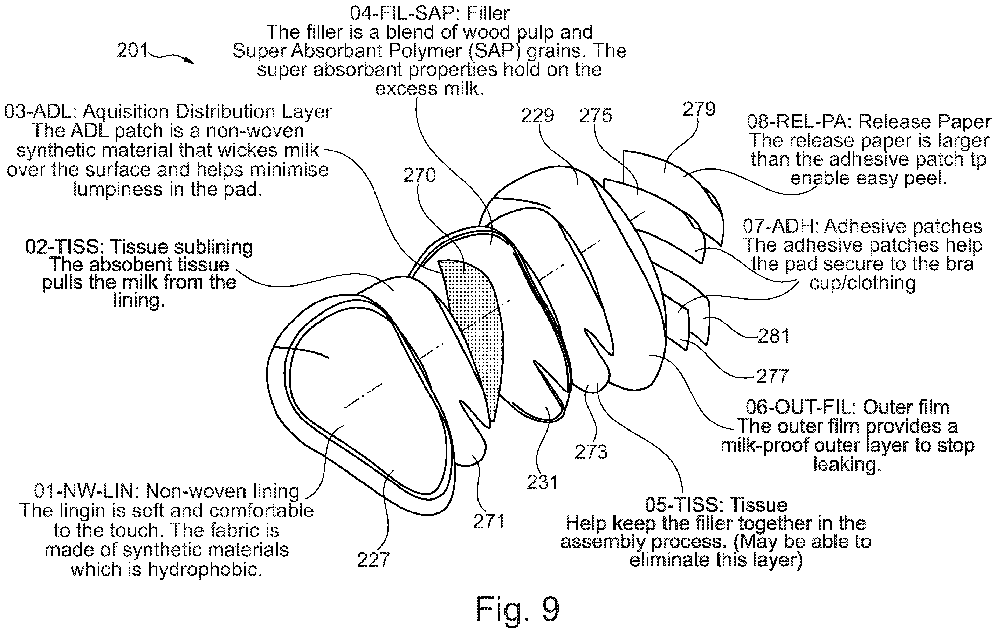

A further example of a breast pad is shown in expanded view in Fig. 9 illustrating additional, optional aspects in a breast pad 201. Thus, in addition to a front sheet 227, back sheet 229, absorbent sheet 231 and liquid distribution portion 270, the breast pad includes a first tissue sheet 271 arranged between the front sheet 227 and the liquid distribution portion 270 to further improve the rate at which liquid permeates from the front sheet 227 to the absorbent sheet 231. Alternatively, the first tissue sheet 271 may be arranged between the liquid distribution portion 270 and the absorbent sheet 231.

A second tissue sheet 273 is an intermediary between the absorbent sheet 231 and the back sheet 229 in order to keep the absorbent sheet 231 in place and provide some protection during the manufacturing process.

On the outer surface of the back sheet two adhesive patches 275, 277 are provided in order to secure the breast pad 201 to the user's clothing while in use. The adhesive patches are provided covered with release papers 279, 281 to protect the adhesive from contamination until the user wants to secure the pad in their clothing.

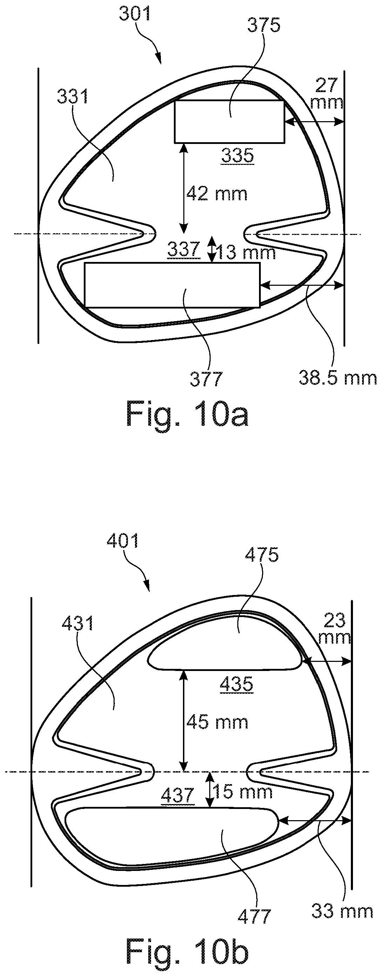

Fig. 10a and 10b further illustrate the adhesive patches (without release papers). Although the patches are shown in front of absorbent sheets, this is only to aid their positioning and, in the final breast pads the adhesive is fixed to the outer surface of the back sheet, as described in the example of Fig. 9.

Thus, in a first example, a breast pad 301 is provided with adhesive in the form of rectangular upper patch 375 and lower patch 377. In use, the breast pads are worn with the lower region 337 generally positioned below the user's nipple and so in daytime use, when the user is usually standing or sitting, gravity ensures that this region of the absorbent material 331 collects more liquid thus making the lower region 337 more susceptible to swelling or distortion. Accordingly, the lower patch 377 is significantly larger than the upper patch 375 and is positioned in front of the lower region 337 of the absorbent material 331 to withstand the swelling or distortion without coming away from the user's clothing and leaking.

Optionally, the patches 475, 477 may be provided as curved or contoured shapes per the example breast pad 401 in Fig. 10b. In this way, the shapes of the patches 475, 477 more accurately match the corresponding shape of the absorbent material 431 positioned directly underneath, further reducing the chance of the adhesive failing as the pad absorbs liquid and distorts or swells.

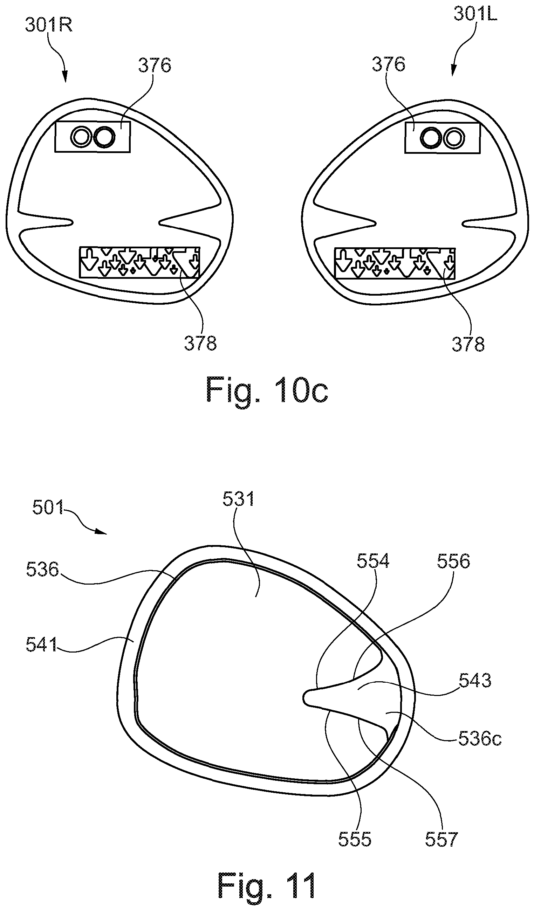

Adhesive patches are typically provided with release paper to protect the adhesive until the pad is used. Because the adhesive is provided in asymmetrical locations, the release papers may be used to indicate orientation of the pads when secured in the clothing of a user. Thus, as provided in Fig. 10c, upper release papers 376 include an indicator in the form of circular symbols printed on their outer surfaces to show which of a pair of breast pads is provided for best fit to the user's left breast 301L and which fits best to their right breast 301R.

In a similar way, the lower release papers 378 have arrow symbols printed on them to act as an indicator and show which edge of the breast pads should be positioned downwards when secured to the clothing for use.

Further optionally, any of the breast pads mentioned herein may include differing absorbent materials in its upper and lower region. Accordingly, in order to address the effect of gravity, the lower region (such as 37, 337, 437) may be provided with increased absorbent capacity compared to the corresponding upper portion. The increased capacity may be provided for by a thicker portion of absorbent material.

Alternatively, or additionally, the absorbent capacity may be increased by modifying the composition of the absorbent material so that it can absorb more in the same thickness of material. In this case, the lower region may include a greater proportion of the SAP compared to other components. As SAP may be the most expensive component of the absorbent material then this arrangement will provide more efficient use of the SAP the region of the breast pad expected to receive more liquid.

It should also be noted that the shaping portions of a pair of opposing edges may not be proximal to the perimeter of the absorbent material. Thus, as shown in the example breast pad 501 of Fig. 11, if the cut-out portion is substantially flared towards the perimeter 536 of the absorbent material 531, then the opposing edges 554, 555 may have respective shaping portions 556, 557 located partially along the edges. In this way, when the shaping portions 556. 557 are brought together to form the shaped configuration then the length of the mouth 536c of the cut-out portion 533 is reduced but not reduced to zero. In this way, the flared opposing edges may provide their own unique contribution to the domed shape because they are able to create an exaggerated deformation of the absorbent material in portions between the shaping portions 556, 557 and the perimeter 536.

Thus, as can be seen from the above examples, the shape of the asymmetric cone (and therefore the shape of the enclosed, irregular volume) can be varied in a number of ways and is thus highly adjustable by varying parameters of the asymmetric footprint of the absorbent material. Hence, not only can the shape of the footprint be adapted, but also the original perimeter length and the cut-out portion dimensions can be varied to provide a range of shaped pads. In this way, a range of breast pads can easily be provided in order to fit to desired bra-cup sizes, or to adapt to regional variations in breast shape, or different shapes for mothers whose children are at different stages of breastfeeding.

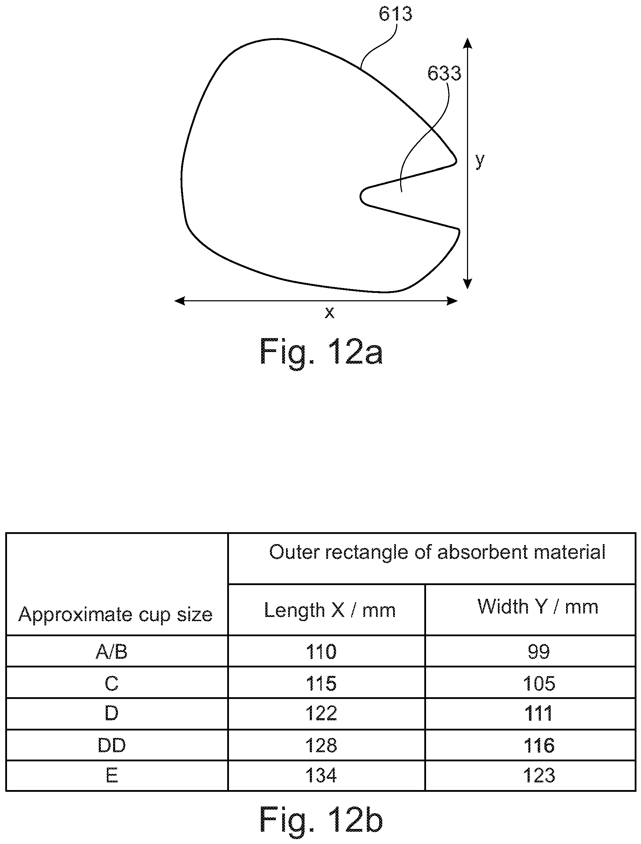

For example, dielines for a range of pads of one asymmetric footprint, covering bra cup sizes A/B to E is described in Figs. 12a & 12b. Thus, the footprint 613 can be utilised in a series of dielines which fit within a rectangular area of length X and width Y. In the example shown, a single cut-out portion 633 is provided. The specific lengths of X and Y for each pad size is listed in the table of Fig. 12b. Further footprint sizes for additional bra cup sizes may be determined.

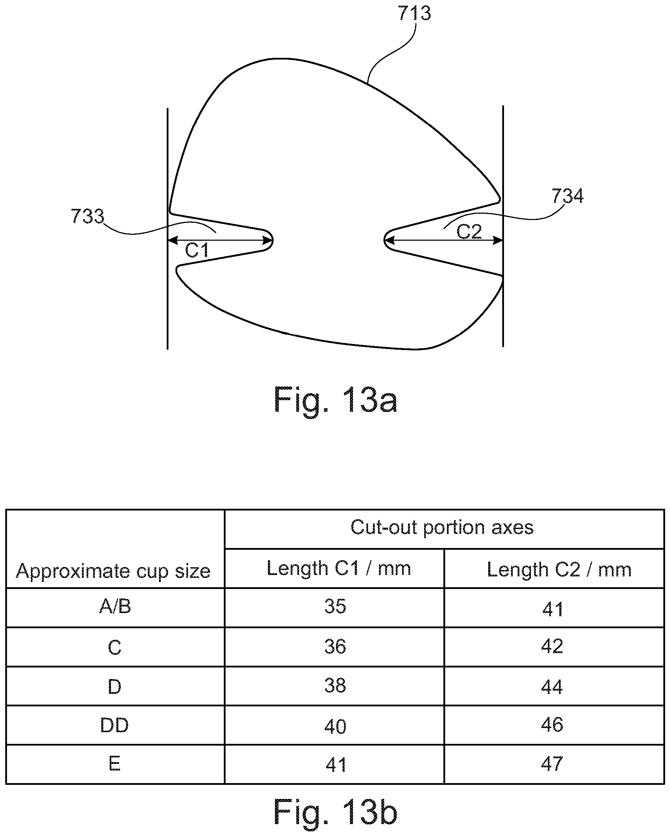

The same footprints may also be used for a range of pads covering the same bra cup sizes but with two cut-out portions (Fig. 13a & 13b). Thus, the footprint includes a series of dielines 713 which fit within the same rectangular areas as series of Fig. 12a. In this case, the cut-out portions 733, 734 include axes of differing lengths C1, C2 as listed in the table of Fig. 13b. In this way, the length C2 that a first cut-out portion extends from the perimeter into the body is greater than the length C1 that a second cut-out portion extends. In this way, the cut-outs allow a flat pad to deform into asymmetrical domes or cup shapes where both the size and the shaping deformation can be chosen according a range of bra-cup sizes. Again, further footprint sizes for additional bra cup sizes may be determined.

The footprints may also be adapted to closely match the contours of either a left or right breast of a nursing mother. Thus, by providing a second shaped breast pad, formed as mirror of the first, it is possible to provide mothers with paired breast pads.

The variability in dome shapes and footprint dielines ensure that nursing mothers can select breasts pads optimised to provide the best fit regardless of their own shape and size. And, by providing breast pads with sizes consistent with bra cup sizes, mothers will be able to select an appropriate size without trial and error. Consequently, it is possible to eliminate the risk of leaking created by using a single-shape pad for every nursing mother because the optimised size and shape the pads are much more likely to correspond to the breast they are used with, and the comfort for the wearer is significantly improved.

It will be appreciated that relative language such as upper, inner, outer, etc. is used herein. This language refers to breast pads as worn by a user while sitting or standing, thereby using the breast pads in an upright orientation as shown in the Figures. This language is used to aid understanding of the disclosed breast pads and should not be considered to limit the present disclosure.

While the breast pads of the present disclosure are described in relation to the examples and embodiments shown in the figures, it will be appreciated that many modifications are described herein. It will also be appreciated that while the examples and embodiments have been described separately, the breast pads of the present disclosure share many common aspects and features, and these features and aspects can be interchangeable.

## Claims

1. A breast pad (50, 101, 201, 301, 401. 501) for nursing mothers comprising:
an absorbent material (31, 231, 331, 431, 531, 831, 931, 1031, 1131) having an asymmetric footprint, a liquid permeable front surface (27, 227) and a liquid impermeable back surface (29, 229), the absorbent material (31, 231, 331, 431, 531, 831, 931, 1031, 1131) arranged to absorb liquid that permeates the front surface (27, 227),
wherein the absorbent material (31, 231, 331, 431, 531, 831, 931, 1031, 1131) comprises a body, a perimeter (36, 136, 536, 836), and at least one cut-out portion (33, 34, 133, 633, 733, 734, 833, 834) extending inwardly from the perimeter (36, 136, 536, 836) into the body,
wherein, the cut-out portion (33, 34, 133, 633, 733, 734, 833, 834) comprises two opposing edges (54, 55, 60, 61, 154, 155, 554, 555, 854, 855,860, 861) each with a shaping portion (56, 57, 62, 63, 156, 156, 556, 557),
wherein the absorbent material (31, 231, 331, 431, 531, 831, 931, 1031, 1131) is adapted such that the perimeter (36, 136, 536, 836) around the absorbent material (31, 231, 331, 431, 531, 831, 931, 1031, 1131) is a first length in a first configuration and, in a second configuration, the two shaping portions (56, 57, 62, 63, 156, 156, 556, 557) are positioned closer together such that the perimeter length is reduced; and
wherein the breast pad (50, 101, 201, 301, 401. 501) comprises an upper portion (35, 335, 435, 835) and a lower portion (37, 337, 437, 837) and wherein in the lower portion (37, 337, 437, 837) the absorbent material (31, 231, 331, 431, 531, 831, 931, 1031, 1131) is adapted to have increased absorbent capacity.

2. A breast pad (50, 101, 201, 301, 401. 501) according to claim 1 wherein in the first configuration the absorbent material (31, 231, 331, 431, 531, 831, 931, 1031, 1131) is planar.

3. A breast pad (50, 101, 201, 301, 401. 501) according to claim 1 or 2, wherein the two shaping portions (56, 57, 62, 63, 156, 156, 556, 557) are provided at or adjacent to the perimeter (36, 136, 536, 836).

4. A breast pad (50, 101, 201, 301, 401. 501) according to any preceding claim, wherein the two opposing edges (54, 55, 60, 61, 154, 155, 554, 555, 854, 855,860, 861) are flared as they approach the perimeter (36, 136, 536, 836).

5. A breast pad (50, 101, 201, 301, 401. 501) according to any preceding claim, wherein at least one of the front surface (27, 227) or back surface (29, 229) forms a deformable sheet that extends between the two opposing edges (54, 55, 60, 61, 154, 155, 554, 555, 854, 855,860, 861) and wherein the deformable sheet is configured to deform in the second configuration and, preferably, wherein the deformable sheet forms at least one pleat in the second configuration.

6. A breast pad (50, 101, 201, 301, 401. 501) according to any preceding claim, comprising securing means configured to position the two shaping portions (56, 57, 62, 63, 156, 156, 556, 557) in the second configuration and, preferably, wherein the securing means is re-positionable.

7. A breast pad (50, 101, 201, 301, 401. 501) according to any preceding claim, wherein at least one of the front surface (27, 227) and the back surface (29, 229) is provided as a sheet which is separable from the absorbent material (31, 231, 331, 431, 531, 831, 931, 1031, 1131) and, preferably, wherein both the front and back surfaces (27, 227, 29, 229) are provided as sheets which extend beyond the perimeter (36, 136, 536, 836) and which together form a sealed edge portion.

8. A breast pad (50, 101, 201, 301, 401. 501) according to any preceding claim, wherein the absorbent material (31, 231, 331, 431, 531, 831, 931, 1031, 1131) comprises more than one cut-out portion (33, 34, 133, 633, 733, 734, 833, 834), each cut-out portion (33, 34, 133, 633, 733, 734, 833, 834) having two opposing edges (54, 55, 60, 61, 154, 155, 554, 555, 854, 855,860, 861) and respective shaping portions (56, 57, 62, 63, 156, 156, 556, 557) and, preferably, wherein the absorbent material (31, 231, 331, 431, 531, 831, 931, 1031, 1131) is adapted such that in the second configuration the respective shaping portions (56, 57, 62, 63, 156, 156, 556, 557) each reduce the perimeter length by different amounts.

9. A breast pad (50, 101, 201, 301, 401. 501) according to claim 8, wherein the length that a first cut-out portion (33, 733, 833) extends from the perimeter (36, 136, 536, 836) into the body is greater than the length that a second cut-out portion (34, 734, 834) extends.

10. A breast pad (50, 101, 201, 301, 401. 501) according to claim 8 or claim 9, wherein each cut-out portion (33, 34, 133, 633, 733, 734, 833, 834) extends from the perimeter (36, 136, 536, 836) into the body along a different axis.

11. A breast pad (50, 101, 201, 301, 401. 501) according to any preceding claim, wherein the absorbent material (31, 231, 331, 431, 531, 831, 931, 1031, 1131) further comprises a liquid distribution portion (70, 270) arranged in contact with the front sheet (27, 227).

12. A breast pad (50, 101, 201, 301, 401. 501) according to any preceding claim, wherein the absorbent sheet (31, 231, 331, 431, 531, 831, 931, 1031, 1131) comprises a plurality of absorbent sub-layers (931a, 931b, 931c, 1031a, 1031b, 1031c, 1131a, 1131b, 1131c) and, preferably, wherein a first absorbent sub-layer (931a, 1031a, 1131a) has different absorbency characteristics to a second absorbent sub-layer (931b, 1031b, 1131b).

13. A breast pad (50, 101, 201, 301, 401. 501) according to claim 12, wherein a first absorbent sub-layer (931a, 931c, 1031b, 1131c) extends to the perimeter (36, 136, 536, 836) and a second absorbent sub-layer (931b, 1031a, 1031c, 1131a, 1131b) does not.

14. A breast pad (50, 101, 201, 301, 401. 501) according to any preceding claim, wherein the back sheet (29, 229) comprises an adhesive portion (275, 277, 375, 377, 475, 477) suitable for securing the pad (50, 101, 201, 301, 401. 501) to the clothing of a user and, preferably, wherein the adhesive portion (275, 277, 375, 377, 475, 477) comprises a release paper (376, 378) which comprises an indicator suitable for indicating orientation of the breast pad (50, 101, 201, 301, 401. 501) when secured to the clothing of a user.

15. A breast pad (50, 101, 201, 301, 401. 501) according to any preceding claim, wherein the breast pad (50, 101, 201, 301, 401. 501) is disposable.

## Patentansprüche

1. Stilleinlage (50, 101, 201, 301, 401. 501) für stillende Mütter, umfassend:
ein Absorptionsmaterial (31, 231, 331, 431, 531, 831, 931, 1031, 1131), das eine asymmetrische Grundfläche, einer flüssigkeitsdurchlässigen Vorderoberfläche (27, 227) und eine flüssigkeitsundurchlässigen Rückoberfläche (29, 229) aufweist, wobei das Absorptionsmaterial (31, 231, 331, 431, 531, 831, 931, 1031, 1131) angeordnet ist, um Flüssigkeit zu absorbieren, die die Vorderoberfläche (27, 227) durchdringt,
wobei das Absorptionsmaterial (31, 231, 331, 431, 531, 831, 931, 1031, 1131) einen Körper, einen Umfang (36, 136, 536, 836) und mindestens einen ausgeschnittenen Abschnitt (33, 34, 133, 633, 733, 734, 833, 834) umfasst, der sich von dem Umfang (36, 136, 536, 836) nach innen in den Körper erstreckt,
wobei der ausgeschnittene Abschnitt (33, 34, 133, 633, 733, 734, 833, 834) zwei gegenüberliegende Kanten (54, 55, 60, 61, 154, 155, 554, 555, 854, 855, 860, 861) umfasst, jeweils mit einem Formabschnitt (56, 57, 62, 63, 156, 156, 556, 557),
wobei das Absorptionsmaterial (31, 231, 331, 431, 531, 831, 931, 1031, 1131) derart angepasst ist, dass der Umfang (36, 136, 536, 836) um das Absorptionsmaterial (31, 231, 331, 431, 531, 831, 931, 1031, 1131) herum eine erste Länge in einer ersten Konfiguration ist und in einer zweiten Konfiguration die zwei Formabschnitte (56, 57, 62, 63, 156, 156, 556, 557) derart näher zusammen positioniert sind, dass die Umfangslänge reduziert ist; und
wobei die Stilleinlage (50, 101, 201, 301, 401. 501) einen oberen Abschnitt (35, 335, 435, 835) und einen unteren Abschnitt (37, 337, 437, 837) umfasst und wobei in dem unteren Abschnitt (37, 337, 437, 837) das Absorptionsmaterial (31, 231, 331, 431, 531, 831, 931, 1031, 1131) angepasst ist, um eine erhöhte Absorptionskapazität aufzuweisen.

2. Stilleinlage (50, 101, 201, 301, 401. 501) nach Anspruch 1, wobei in der ersten Konfiguration das Absorptionsmaterial (31, 231, 331, 431, 531, 831, 931, 1031, 1131) planar ist.

3. Stilleinlage (50, 101, 201, 301, 401. 501) nach Anspruch 1 oder 2, wobei die zwei Formabschnitte (56, 57, 62, 63, 156, 156, 556, 557) an dem oder angrenzend an den Umfang (36, 136, 536, 836) bereitgestellt sind.

4. Stilleinlage (50, 101, 201, 301, 401. 501) nach einem der vorstehenden Ansprüche, wobei die zwei gegenüberliegenden Kanten (54, 55, 60, 61, 154, 155, 554, 555, 854, 855, 860, 861) ausgestellt sind, wenn sie sich dem Umfang (36, 136, 536, 836) nähern.

5. Stilleinlage (50, 101, 201, 301, 401. 501) nach einem der vorstehenden Ansprüche, wobei mindestens eine der Vorderoberfläche (27, 227) oder der Rückoberfläche (29, 229) eine verformbare Lage ausbildet, die sich zwischen den zwei gegenüberliegenden Kanten (54, 55, 60, 61, 154, 155, 554, 555, 854, 855, 860, 861) erstreckt, und wobei die verformbare Lage konfiguriert ist, um sich in der zweiten Konfiguration zu verformen, und vorzugsweise wobei die verformbare Lage in der zweiten Konfiguration mindestens eine Falte ausbildet.

6. Stilleinlage (50, 101, 201, 301, 401. 501) nach einem der vorstehenden Ansprüche, umfassend Sicherungsmittel, die konfiguriert sind, um die zwei Formabschnitte (56, 57, 62, 63, 156, 156, 556, 557) in der zweiten Konfiguration zu positionieren, und vorzugsweise wobei die Sicherungsmittel neu positionierbar sind.

7. Stilleinlage (50, 101, 201, 301, 401. 501) nach einem der vorstehenden Ansprüche, wobei mindestens eine der Vorderoberfläche (27, 227) und der Rückoberfläche (29, 229) als eine Lage bereitgestellt wird, die von dem Absorptionsmaterial (31, 231, 331, 431, 531, 831, 931, 1031, 1131) trennbar ist, und vorzugsweise wobei sowohl die Vorder- als auch die Rückoberfläche (27, 227, 29, 229) als Lagen bereitgestellt werden, die sich über den Umfang (36, 136, 536, 836) hinaus erstrecken und die zusammen einen abgedichteten Kantenabschnitt ausbilden.

8. Stilleinlage (50, 101, 201, 301, 401. 501) nach einem der vorstehenden Ansprüche, wobei das Absorptionsmaterial (31, 231, 331, 431, 531, 831, 931, 1031, 1131) mehr als einen ausgeschnittenen Abschnitt (33, 34, 133, 633, 733, 734, 833, 834) umfasst, wobei jeder ausgeschnittene Abschnitt (33, 34, 133, 633, 733, 734, 833, 834) zwei gegenüberliegende Kanten (54, 55, 60, 61, 154, 155, 554, 555, 854, 855, 860, 861) und jeweilige Formabschnitte (56, 57, 62, 63, 156, 156, 556, 557) aufweist und vorzugsweise wobei das Absorptionsmaterial (31, 231, 331, 431, 531, 831, 931, 1031, 1131) derart angepasst ist, dass in der zweiten Konfiguration die jeweiligen Formabschnitte (56, 57, 62, 63, 156, 156, 556, 557) die Umfangslänge jeweils um unterschiedliche Beträge reduzieren.

9. Stilleinlage (50, 101, 201, 301, 401. 501) nach Anspruch 8, wobei die Länge, um die sich ein erster ausgeschnittener Abschnitt (33, 733, 833) von dem Umfang (36, 136, 536, 836) in den Körper hinein erstreckt, größer als die Länge ist, um die sich ein zweiter ausgeschnittener Abschnitt (34, 734, 834) erstreckt.

10. Stilleinlage (50, 101, 201, 301, 401. 501) nach Anspruch 8 oder 9, wobei sich jeder ausgeschnittene Abschnitt (33, 34, 133, 633, 733, 734, 833, 834) von dem Umfang (36, 136, 536, 836) entlang einer unterschiedlichen Achse in den Körper hinein erstreckt.

11. Stilleinlage (50, 101, 201, 301, 401. 501) nach einem der vorstehenden Ansprüche, wobei das Absorptionsmaterial (31, 231, 331, 431, 531, 831, 931, 1031, 1131) ferner einen Flüssigkeitsverteilungsabschnitt (70, 270) umfasst, der in Kontakt mit der Vorderlage (27, 227) angeordnet ist.

12. Stilleinlage (50, 101, 201, 301, 401. 501) nach einem der vorstehenden Ansprüche, wobei die Absorptionslage (31, 231, 331, 431, 531, 831, 931, 1031, 1131) eine Vielzahl von Absorptionsunterschichten (931a, 931b, 931c, 1031a, 1031b, 1031c, 1131a, 1131b, 1131c) umfasst und vorzugsweise wobei eine erste Absorptionsunterschicht (931a, 1031a, 1131a) unterschiedliche Absorptionseigenschaften als eine zweite Absorptionsunterschicht (931b, 1031b, 1131b) aufweist.

13. Stilleinlage (50, 101, 201, 301, 401. 501) nach Anspruch 12, wobei sich eine erste Absorptionsunterschicht (931a, 931c, 1031b, 1131c) zu dem Umfang (36, 136, 536, 836) erstreckt und eine zweite Absorptionsunterschicht (931b, 1031a, 1031c, 1131a, 1131b) nicht.

14. Stilleinlage (50, 101, 201, 301, 401. 501) nach einem der vorstehenden Ansprüche, wobei die Rücklage (29, 229) einen Klebeabschnitt (275, 277, 375, 377, 475, 477) umfasst, der zum Sichern der Einlage (50, 101, 201, 301, 401. 501) an der Kleidung eines Benutzers geeignet ist, und vorzugsweise wobei der Klebeabschnitt (275, 277, 375, 377, 475, 477) ein Trennpapier (376, 378) umfasst, das einen Indikator umfasst, der zum Anzeigen einer Ausrichtung der Stilleinlage (50, 101, 201, 301, 401. 501) geeignet ist, wenn es an der Kleidung eines Benutzers gesichert ist.

15. Stilleinlage (50, 101, 201, 301, 401. 501) nach einem der vorstehenden Ansprüche, wobei die Stilleinlage (50, 101, 201, 301, 401. 501) ein Einwegartikel ist.

## Revendications

1. Compresse mammaire (50, 101, 201, 301, 401. 501) pour les mères allaitantes comprenant :
un matériau absorbant (31, 231, 331, 431, 531, 831, 931, 1031, 1131) ayant une empreinte asymétrique, une surface avant perméable aux liquides (27, 227) et une surface arrière imperméable aux liquides (29, 229), le matériau absorbant (31, 231, 331, 431, 531, 831, 931, 1031, 1131) étant agencé pour absorber les liquides qui traversent la surface avant (27, 227),
dans laquelle le matériau absorbant (31, 231, 331, 431, 531, 831, 931, 1031, 1131) comprend un corps, un périmètre (36, 136, 536, 836) et au moins une partie découpée (33, 34, 133, 633, 733, 734, 833, 834) s'étendant vers l'intérieur du le périmètre (36, 136, 536, 836) dans le corps,
dans laquelle, la partie découpée (33, 34, 133, 633, 733, 734, 833, 834) comprend deux bords opposés (54, 55, 60, 61, 154, 155, 554, 555, 854, 855, 860, 861) ayant chacun une partie de mise en forme (56, 57, 62, 63, 156, 156, 556, 557),
dans laquelle le matériau absorbant (31, 231, 331, 431, 531, 831, 931, 1031, 1131) est adapté de telle sorte que le périmètre (36, 136, 536, 836) autour du matériau absorbant (31, 231, 331, 431, 531, 831, 931, 1031, 1131) est d'une première longueur dans une première configuration et, dans une seconde configuration, les deux parties de mise en forme (56, 57, 62, 63, 156, 156, 556, 557) sont positionnées plus près l'une de l'autre de telle sorte que la longueur du périmètre est réduite ; et
dans laquelle la compresse mammaire (50, 101, 201, 301, 401. 501) comprend une partie supérieure (35, 335, 435, 835) et une partie inférieure (37, 337, 437, 837) et dans laquelle, dans la partie inférieure (37, 337, 437, 837), le matériau absorbant (31, 231, 331, 431, 531, 831, 931, 1031, 1131) est adapté pour avoir une capacité d'absorption accrue.

2. Compresse mammaire (50, 101, 201, 301, 401. 501) selon la revendication 1, dans laquelle, dans la première configuration, le matériau absorbant (31, 231, 331, 431, 531, 831, 931, 1031, 1131) est plan.

3. Compresse mammaire (50, 101, 201, 301, 401. 501) selon la revendication 1 ou 2, dans laquelle les deux parties de mise en forme (56, 57, 62, 63, 156, 156, 556, 557) sont prévues au niveau du périmètre (36, 136, 536, 836) ou à proximité de celui-ci.

4. Compresse mammaire (50, 101, 201, 301, 401. 501) selon l'une quelconque revendication précédente, dans laquelle les deux bords opposés (54, 55, 60, 61, 154, 155, 554, 555, 854, 855, 860, 861) sont évasés à l'approche du périmètre (36, 136, 536, 836).

5. Compresse mammaire (50, 101, 201, 301, 401. 501) selon l'une quelconque revendication précédente, dans laquelle au moins l'une parmi la surface avant (27, 227) ou la surface arrière (29, 229) forme une feuille déformable qui s'étend entre les deux bords opposés (54, 55, 60, 61, 154, 155, 554, 555, 854, 855, 860, 861) et dans laquelle la feuille déformable est conçue pour se déformer dans la seconde configuration et, de préférence, dans laquelle la feuille déformable forme au moins un pli dans la seconde configuration.

6. Compresse mammaire (50, 101, 201, 301, 401. 501) selon l'une quelconque revendication précédente, comprenant un moyen de fixation conçu pour positionner les deux parties de mise en forme (56, 57, 62, 63, 156, 156, 556, 557) dans la seconde configuration et, de préférence, dans laquelle le moyen de fixation est repositionnable.

7. Compresse mammaire (50, 101, 201, 301, 401. 501) selon l'une quelconque revendication précédente, dans laquelle au moins l'une parmi la surface avant (27, 227) et la surface arrière (29, 229) est fournie sous forme de feuille qui est séparable du matériau absorbant (31, 231, 331, 431, 531, 831, 931, 1031, 1131) et, de préférence, dans laquelle les surfaces avant et arrière (27, 227, 29, 229) sont toutes deux fournies sous forme de feuilles qui s'étendent au-delà du périmètre (36, 136, 536, 836) et qui, ensemble, forment une partie de bord scellée.

8. Compresse mammaire (50, 101, 201, 301, 401. 501) selon l'une quelconque revendication précédente, dans laquelle le matériau absorbant (31, 231, 331, 431, 531, 831, 931, 1031, 1131) comprend plus d'une partie découpée (33, 34, 133, 633, 733, 734, 833, 834), chaque partie découpée (33, 34, 133, 633, 733, 734, 833, 834) ayant deux bords opposés (54, 55, 60, 61, 154, 155, 554, 555, 854, 855, 860, 861) et des parties de mise en forme (56, 57, 62, 63, 156, 156, 556, 557) respectives et, de préférence, dans laquelle le matériau absorbant (31, 231, 331, 431, 531, 831, 931, 1031, 1131) est adapté de telle sorte que, dans la seconde configuration, les parties de mise en forme (56, 57, 62, 63, 156, 156, 556, 557) respectives réduisent chacune la longueur du périmètre dans des proportions différentes.

9. Compresse mammaire (50, 101, 201, 301, 401. 501) selon la revendication 8, dans laquelle la longueur sur laquelle une première partie découpée (33, 733, 833) s'étend du périmètre (36, 136, 536, 836) dans le corps est supérieure à la longueur sur laquelle une seconde partie découpée (34, 734, 834) s'étend.

10. Compresse mammaire (50, 101, 201, 301, 401. 501) selon la revendication 8 ou la revendication 9, dans laquelle chaque partie découpée (33, 34, 133, 633, 733, 734, 833, 834) s'étend du périmètre (36, 136, 536, 836) dans le corps le long d'un axe différent.

11. Compresse mammaire (50, 101, 201, 301, 401. 501) selon l'une quelconque revendication précédente, dans laquelle le matériau absorbant (31, 231, 331, 431, 531, 831, 931, 1031, 1131) comprend en outre une partie de distribution de liquide (70, 270) agencée en contact avec la feuille avant (27, 227).

12. Compresse mammaire (50, 101, 201, 301, 401. 501) selon l'une quelconque revendication précédente, dans laquelle la feuille absorbante (31, 231, 331, 431, 531, 831, 931, 1031, 1131) comprend une pluralité de sous-couches absorbantes (931a, 931b, 931c, 1031a, 1031b, 1031c, 1131a, 1131b, 1131c) et, de préférence, dans laquelle une première sous-couche absorbante (931a, 1031a, 1131a) a des caractéristiques d'absorption différentes de celles d'une seconde sous-couche absorbante (931b, 1031b, 1131b).

13. Compresse mammaire (50, 101, 201, 301, 401. 501) selon la revendication 12, dans laquelle une première sous-couche absorbante (931a, 931c, 1031b, 1131c) s'étend jusqu'au périmètre (36, 136, 536, 836) et une seconde sous-couche absorbante (931b, 1031a, 1031c, 1131a, 1131b) ne s'étend pas.

14. Compresse mammaire (50, 101, 201, 301, 401. 501) selon l'une quelconque revendication précédente, dans laquelle la feuille arrière (29, 229) comprend une partie adhésive (275, 277, 375, 377, 475, 477) apte à fixer la compresse (50, 101, 201, 301, 401. 501) aux vêtements d'un utilisateur et, de préférence, dans laquelle la partie adhésive (275, 277, 375, 377, 475, 477) comprend un papier de libération (376, 378) qui comprend un indicateur apte à indiquer une orientation de la compresse mammaire (50, 101, 201, 301, 401. 501) lorsqu'elle est fixée aux vêtements d'un utilisateur.

15. Compresse mammaire (50, 101, 201, 301, 401. 501) selon l'une quelconque revendication précédente, dans laquelle la compresse mammaire (50, 101, 201, 301, 401. 501) est jetable.
